# FASCICULE DE BREVET EUROPEEN

(11) **EP 2 268 236 B1**
(45) Date de publication et mention de la délivrance du brevet: **26.02.2014**
(21) Numéro de dépôt: 09742324.8
(22) Date de dépôt: 10.04.2009
(51) Int. Cl.: A61F 5/00, A61F 2/00

(54) **ANNEAU GASTRIQUE AVEC POCHES BASCULANTES**
MAGENBAND MIT UMSCHALTUNGSTASCHEN
GASTRIC RING WITH SWITCHING POCKETS

(30) Priorité: 14.04.2008 FR 0802053
(43) Date de publication de la demande: 05.01.2011
(73) Titulaire: Compagnie Europeenne d'Etude et de Recherche de Dispositifs pour l'Implantation par Laparoscopie, 38200 Vienne (FR)
(72) Inventeur: RICOL, Jean-Paul, Gilbert, F-69210 Saint Germain Sur L'arbresle (FR); PAGANON, Pascal, F-69360 Serezin Du Rhône (FR)
(74) Mandataire: Guérin, Jean-Philippe
(86) Numéro de dépôt international: PCT/FR2009/050676
(87) Numéro de publication internationale: WO 2009/136126

(56) Documents cités:
- EP-A- 0 028 962
- EP-A- 1 342 458
- US-A- 4 019 499

## Description

### DOMAINE TECHNIQUE

La présente invention se rapporte au domaine technique général des implants chirurgicaux destinés à être disposés dans le corps d'un patient autour d'un organe biologique constituant une poche ou un conduit, et notamment aux anneaux gastriques permettant de réaliser une constriction de l'estomac dans le cadre d'un traitement de l'obésité.

La présente invention concerne plus particulièrement un anneau chirurgical implantable destiné à être placé autour d'un organe biologique constituant une poche ou un conduit, en vue de modifier la section de passage dudit organe biologique, ledit anneau comportant au moins un premier organe d'appui conçu pour venir en appui contre l'organe biologique et apte à s'étendre vers l'intérieur de l'anneau selon une première direction sensiblement radiale.

### TECHNIQUE ANTERIEURE

Le document EP0028962 décrit un sphincter artificiel formé d'une bande flexible présentant une série de chambre. Les chambres ont des parois hermétiques flexibles et st interconnectées. Une des chambres est connectée à un conduit à travers lequel on introduit ou retire du fluide au moyen d'une pompe. On réalise ainsi le remplissage ou le vidage des différentes chambres et ainsi leur gonflage et dégonflabe. La bande est disposée autour de l'intestin à proximité du stoma, sur la face extérieure de celui-ci. La pompe est actionnée manuellement. Lors du gonflement, les chambres se déforment pour occuper un espace intermédiaire entre elles jusqu'à venir en contact.

Il est connu de pratiquer des interventions chirurgicales sur des patients atteints d'obésité sévère qui, en raison de leur surpoids, sont exposés non seulement à une gêne physique, mais également à une charge psychologique, ainsi qu'à des maladies annexes, tel que le diabète, les maladies cardiovasculaires, ou encore l'arthrite sévère.

En particulier, une technique connue consiste à réaliser une constriction gastrique permettant de réduire la taille de l'estomac, et par conséquent la prise d'aliments.

A cet effet, on recourt fréquemment à l'utilisation d'anneaux de gastroplastie implantés autour de l'estomac du patient en vue de réduire le volume ainsi que le diamètre de passage (stoma) de celui-ci.

Généralement, de tels anneaux de gastroplastie comportent une bande souple réalisée en matériau élastomère et destinée à être fermée vers ses deux extrémités par des moyens de fermeture appropriés afin d'enserrer l'estomac.

En outre, les anneaux connus comportent usuellement une chambre de compression annulaire située sur la face interne de la bande souple et dont le volume est réglable par adjonction ou retrait d'un fluide de remplissage.

De manière générale, les anneaux implantables connus donnent satisfaction, mais souffrent parfois de certains inconvénients.

En particulier, le gonflage de la chambre de compression provoque parfois une déformation irrégulière de la surface de celle-ci, et notamment la formation de plis qui sont susceptibles d'endommager la paroi de l'estomac par pincement ou abrasion.

De surcroît, le gonflage de la chambre de compression peut engendrer une certaine fatigue du matériau constitutif de la paroi de ladite chambre, notamment par distension de cette dernière, ce qui tend à réduire la longévité de l'anneau.

Enfin, il arrive parfois que les anneaux de l'art antérieur se déplacent accidentellement sous l'effet des mouvements naturels de l'estomac, de telle sorte que l'anneau perd en tout ou partie son efficacité thérapeutique et qu'une nouvelle intervention chirurgicale doit être pratiquée.

### EXPOSE DE L'INVENTION

Les objets assignés à la présente invention visent par conséquent à remédier aux inconvénients susmentionnés et à proposer un nouvel anneau chirurgical implantable, notamment gastrique, qui possède de bonnes capacités d'adaptation à des conditions d'utilisation variées, tout en étant particulièrement respectueux de l'organe biologique traité.

Un autre objet assigné à l'invention vise à proposer un nouvel anneau chirurgical implantable qui possède une structure à la fois simple et robuste lui conférant une importante longévité.

Un autre objet assigné à l'invention vise à proposer un nouvel anneau chirurgical implantable qui présente un comportement dynamique prévisible, reproductible, et qui soit particulièrement stable une fois implanté.

Les objets assignés à l'invention sont atteints à l'aide d'un anneau chirurgical implantable destiné à être placé autour d'un organe biologique constituant une poche ou un conduit, en s'enroulant autour d'un axe moyen d'extension (X-X'), en vue de modifier la section de passage dudit organe biologique, ledit anneau comportant au moins un premier organe d'appui conçu pour venir en appui contre l'organe biologique et apte à s'étendre vers l'intérieur de l'anneau selon une première direction sensiblement radiale, ledit anneau étant caractérisé en ce que ledit premier organe d'appui est délimité par une première membrane pourvue d'une structure déformable qui est asymétrique vis-à-vis de ladite première direction et qui est conçue pour provoquer, lors de l'expansion du premier organe d'appui selon la première direction sensiblement radiale, la migration conjointe d'au moins une partie de la première membrane d'un côté à l'autre d'un plan médian audit premier organe d'appui et normal à l'axe d'extension (X-X').

### DESCRIPTIF SOMMAIRE DES DESSINS

D'autres objets, caractéristiques et avantages de l'invention apparaîtront plus en détail à la lecture de la description qui suit, ainsi qu'à l'aide des dessins annexés, fournis à titre purement illustratif et non limitatif, parmi lesquels :
- La figure 1 illustre, selon une vue en perspective, une variante de réalisation d'anneau conforme à l'invention en configuration fermée.
- La figure 2 illustre, selon une vue en perspective avec arrachement de matière, une portion de l'anneau illustré à la figure 1.
- La figure 3 illustre, selon une vue schématique en coupe transversale, une section de l'anneau représenté sur les figures 1 et 2, en configuration contractée d'une part et en configuration déployée d'autre part.

### MEILLEURE MANIERE DE REALISER L'INVENTION

La présente invention concerne un anneau chirurgical implantable 1 qui est destiné à être placé autour d'un organe biologique (non représenté) constituant une poche ou un conduit en vue de modifier la section de passage dudit organe biologique.

A cet effet, l'anneau 1 comporte de préférence une bande souple 2 pourvue de moyens de jonction 3, 4 qui sont conçus pour fermer ladite bande souple 2 sur elle-même, de manière à ce que celle-ci forme une boucle close autour de l'organe biologique.

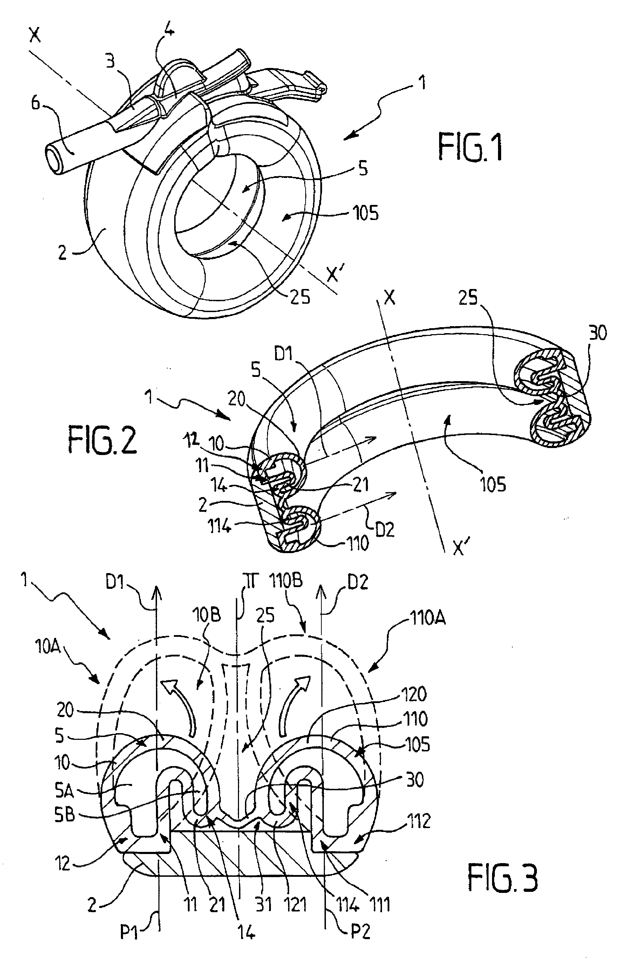

Plus particulièrement, la bande souple 2, et plus globalement l'anneau 1, sont conçus pour s'enrouler géométriquement autour d'un axe moyen d'extension (X-X'), tel que cela est représenté sur les figures 1 et 2, ledit axe d'extension (X-X') coïncidant avantageusement, après implantation, avec la direction principale d'extension du conduit ou de la poche formé par l'organe biologique.

Avantageusement, la bande souple 2 est suffisamment flexible pour passer d'une configuration ouverte (non représentée) à une configuration fermée, représentée sur la figure 1, dans laquelle les moyens de jonction 3, 4 coopèrent, par exemple par encliquetage, pour fermer ladite bande souple 2 sur elle-même sensiblement au niveau de ses extrémités.

A ce titre, lesdits moyens de jonction 3, 4 peuvent comporter un ergot 3 faisant saillie sur une tige liée à la première extrémité et venant en appui contre le rebord d'un manchon 4 fixé à la seconde extrémité de ladite bande souple 2.

De préférence, la bande souple 2, ou « *structure porteuse* », est sensiblement inextensible selon sa direction principale d'extension, de telle sorte qu'une fois fermée, elle forme une structure porteuse dont le périmètre présente une longueur sensiblement invariante.

L'anneau conforme à l'invention constitue préférentiellement un anneau de gastroplastie destiné au traitement de l'obésité et conçu pour être implanté autour de l'estomac afin de réduire le diamètre de l'ouverture du stoma, ou encore pour être implanté autour de l'oesophage.

Bien entendu, l'invention n'est nullement limitée à cette application, et concerne notamment les anneaux chirurgicaux formant des sphincters artificiels adaptés pour le traitement de l'incontinence urinaire ou fécale, ou encore les anneaux utilisés pour la régulation du débit sanguin.

Selon sa destination, l'anneau chirurgical 1 sera bien entendu adapté aux dimensions, à l'environnement et à la sensibilité de l'organe biologique concerné par la constriction, tel que vessie, urètre, intestin, artère, veine, etc.

Avantageusement, l'anneau chirurgical 1 comporte au moins un premier organe d'appui 5 qui est conçu pour venir en appui contre l'organe biologique et qui est apte à s'étendre vers l'intérieur de l'anneau 1 selon une première direction D1 sensiblement radiale.

Ainsi, ledit premier organe d'appui 5 fait avantageusement saillie vers l'intérieur de la boucle close formée par l'anneau en configuration fermée, et est à même de subir une expansion sensiblement radiale centripète, en direction de l'axe d'extension (X-X'), de telle sorte que le praticien puisse ajuster, et notamment restreindre, la lumière de l'anneau 1 à travers laquelle passe l'organe biologique.

Ainsi, ledit premier organe d'appui 5, et plus globalement l'anneau 1, peuvent passer d'une configuration « *contractée* », dans laquelle le premier organe d'appui 5 se trouve éloigné de l'axe (X-X') à une distance initiale de celui-ci, à une configuration « *déployée* » dans laquelle le premier organe d'appui 5 est rapproché de l'axe (X-X'), à une distance inférieure à sa distance initiale, et réciproquement.

De façon particulièrement préférentielle, le premier moyen d'appui 5 peut présenter une structure sensiblement annulaire d'axe (X-X'), et s'étendre sensiblement sur toute la longueur de la bande souple 2 de sorte à pouvoir exercer une constriction ajustable de l'organe biologique.

Bien entendu, la nature, la forme et les dimensions du premier organe d'appui 5 ne sont nullement limitées.

En particulier, il est envisageable que ledit premier organe d'appui soit réalisé par un boudin de matière plein, ou encore par une cavité scellée remplie d'un fluide quelconque.

Toutefois, de façon particulièrement préférentielle, ledit premier organe d'appui 5 sera formé par une première poche gonflable destinée à être remplie par un fluide de remplissage.

Par commodité de description, ledit premier organe d'appui sera assimilé à une première poche gonflable dans ce qui suit.

Avantageusement, ladite première poche gonflable 5 communique, par exemple au moyen d'un conduit 6 de type cathéter, avec un moyen de transfert de fluide déporté (non représenté), tel qu'une seringue ou un réservoir implanté, de telle sorte qu'il est possible d'ajuster librement la quantité de fluide de remplissage contenu dans ladite poche, par injection ou ponction.

Ainsi, le premier organe d'appui 5 est susceptible de passer d'une configuration contractée dans laquelle il contient un volume minimal de fluide, et qui est représentée en traits pleins sur la figure 3, à une configuration déployée dans laquelle il contient un volume de fluide plus important, et qui est représentée en traits pointillés sur cette même figure 3. En outre, ledit premier moyen d'appui 5 peut avantageusement occuper toute configuration intermédiaire entre les deux configurations contractée et déployée, de telle sorte que l'on peut procéder à un réglage sensiblement continu de la constriction de l'organe biologique dans une plage prédéterminée.

Selon une caractéristique importante de l'invention, le premier organe d'appui 5 est délimité par une première membrane 10, ladite première membrane 10 étant pourvue d'une structure déformable qui est asymétrique vis-à-vis de la première direction D1 selon laquelle ledit premier organe d'appui 5 est susceptible de s'étendre.

Par *« pourvue d'une structure déformable asymétrique »,* on indique que la première membrane 10 présente, en particulier lorsqu'elle se trouve au repos, en configuration contractée, une constitution différente dans sa partie 10A située « *à gauche »* de la première direction D1 sur la figure 3, de celle qu'elle présente dans la partie 10B située « *à droite »* de cette même première direction D1.

Ainsi, le comportement en déformation de ladite membrane, sous l'effet du gonflage par ajout de fluide de remplissage (ou inversement, du dégonflage par purge dudit fluide) n'est pas symétrique de part et d'autre de la ligne de séparation matérialisant ladite première direction D1.

Il en résulte par conséquent une déformation asymétrique de la première poche gonflable 5, qui peut avantageusement permettre de ré-agencer la matière constitutive de ladite première membrane 10 lors de son déploiement, et ainsi limiter considérablement la probabilité de formation de plis susceptibles de provoquer le pincement de la paroi de l'organe biologique.

Ainsi, en créant volontairement une déformation asymétrique contrôlée du premier organe d'appui 5, on confère à l'anneau un comportement particulièrement atraumatique.

Bien entendu, les différences structurelles entre la partie *« gauche »* 10A et la partie « *droite »* 10B de la première membrane 10 ne sont nullement limitées.

Ainsi, selon une variante de réalisation préférentielle représentée sur les figures 2 et 3, ces différences peuvent être d'ordre géométrique, la membrane 10 présentant un agencement et une répartition spatiale différents dans sa partie gauche 10A et sa partie droite 10B.

Selon une autre variante de réalisation (non représentée), ces différences peuvent résider dans la nature des matériaux constitutifs de chacune desdites parties gauche et droite, et plus particulièrement dans la rigidité intrinsèque desdits matériaux, de sorte à favoriser la déformation dans la partie droite 10B au moyen d'un matériau souple, tandis qu'on la limite dans la partie gauche 10A à l'aide d'un matériau plus rigide, ou inversement.

De préférence, la structure déformable de la première membrane 10 est conçue pour provoquer, lors de l'expansion du premier organe d'appui 5 selon la première direction D1 sensiblement radiale, la migration conjointe d'au moins une partie de la première membrane 10 d'un côté à l'autre d'un plan P1 médian audit premier organe d'appui 5 et normal à l'axe d'extension (X-X').

En d'autres termes, le déplacement radial centripète de la partie saillante de la première membrane 10 s'accompagne avantageusement d'une certaine déviation latérale d'au moins une portion de ladite première membrane 10, de telle sorte que l'on assiste à un transfert de la matière constitutive de ladite première membrane 10 d'un côté à l'autre du plan médian P1, selon une composante transverse à la première direction D1 et sensiblement portée par la direction de l'axe d'extension (X-X'), c'est-à-dire qu'au moins une partie de ladite première membrane 10 qui se situe initialement dans la partie droite 10B de cette dernière, lorsque le premier organe d'appui 5 se trouve en configuration contractée, se déplace globalement jusqu'à rejoindre la partie gauche 10A située de l'autre côté du plan médian P1 lorsque le premier organe d'appui 5 passe de sa configuration contractée à sa configuration déployée sous l'effet du gonflage.

Avantageusement, ce ré-agencement de la répartition spatiale de la matière constitutive de la première membrane 10 permet d'obtenir un certain effet de roulement de ladite première membrane 10 le long de la surface de l'organe biologique, ce qui limite considérablement les risques d'abrasion ou de pincement de la paroi de ce dernier.

De préférence, le premier organe d'appui 5 est fixé à la structure porteuse 2 respectivement au niveau d'une première zone de jonction latérale 11 et d'une seconde zone de jonction latérale 12 qui sont distantes l'une de l'autre, la première membrane 10 étant alors rabattue sur elle-même du côté de la première zone de jonction latérale 11, lorsque le premier organe d'appui 5 se trouve en configuration contractée, de sorte à former une réserve de matière 14 située sensiblement à l'opposé de la seconde jonction latérale 12.

En d'autres termes, tel que cela est illustré sur les figures 2 et 3, la première membrane 10 présente avantageusement un excédent de matière située dans sa partie droite 10B, de sorte à pouvoir transférer une partie de ladite matière d'un bord à l'autre, en l'occurrence de la droite vers la gauche, du plan médian P1 lors du déploiement du premier organe d'appui 5.

De préférence, la première et la seconde zone de jonction forment des lignes sensiblement circulaires et parallèles, d'axe (X-X'), par lesquelles la base de la première poche gonflable 5 est continûment fixée à la structure porteuse 2

Avantageusement, la première et la seconde zone de jonction latérale 11, 12 forment des points fixes sensiblement invariants, par rapport auxquels s'opère le ré-agencement de la membrane 10 lors du gonflage ou de la vidange de la première poche gonflable 5.

Il est remarquable que la réserve de matière 14 conforme à l'invention autorise le déploiement du premier organe d'appui 5 sensiblement sans créer de tension importante au niveau de la première membrane 10, ce qui permet d'éviter toute distension ou toute craquelure accidentelle de cette dernière.

On obtient ainsi une structure particulièrement robuste et peu sujette à la fatigue, de telle sorte que l'anneau 1 possède une importante longévité.

De préférence, tel que cela est illustré sur la figure 3, la première membrane 10 comprend, lorsque le premier organe d'appui 5 se trouve en configuration contractée, un premier tronçon 20 dit « lisse » relié à la seconde zone de jonction latérale 12, et un second tronçon 21 dit « plissé » relié à la première zone de jonction latérale 11, le premier tronçon lisse 20 rejoignant le second tronçon plissé 21 et formant un dôme qui recouvre à la fois ledit second tronçon plissé 21 et la première zone de jonction latérale 11.

Avantageusement, ce second tronçon plissé 21 constitue la réserve de matière 14 susmentionnée.

En outre, selon une caractéristique préférentielle qui peut constituer une invention à part entière, la membrane 10 présente ainsi avantageusement une structure asymétrique apte à se dérouler ou au contraire à s'enrouler sur elle-même, notamment selon un mouvement latéral de redressement, respectivement de fléchissement, porté par l'axe (X-X'), lors du déploiement radial centripète, respectivement du retrait radial centrifuge, de l'organe d'appui 5.

De préférence, le premier tronçon lisse 20 forme un dôme régulier à la surface lisse et convexe par rapport à l'intérieur de l'anneau 1, notamment hémicylindrique, qui sépare l'organe biologique de la zone plissée du second tronçon 21, de telle sorte que cette dernière n'est pas susceptible de venir au contact direct de la paroi de l'organe biologique lorsque l'anneau se trouve en configuration contractée. On évite ainsi avantageusement le pincement de l'organe biologique au niveau du second tronçon 21.

Par ailleurs, le premier tronçon 20 et le second tronçon 21 sont de préférence venus de matière de sorte à former une unique première membrane 10 monobloc.

Bien entendu, la forme, l'agencement et le nombre des plis du second tronçon 21 ne sont nullement limités.

Toutefois, selon une variante de réalisation préférentielle qui peut constituer une invention en tant que telle, les couches formées par les plis du second tronçon 21 sont sensiblement empilées selon des plans normaux à l'axe moyen d'extension (X-X'), c'est-à-dire s'étendent sensiblement parallèlement au plan médian P1 du premier organe d'appui 5.

Avantageusement, cet agencement permet de stocker la réserve de matière 14 dans l'espace délimité sous le premier tronçon lisse 20, entre ledit premier tronçon 20 et la structure porteuse 2 formant la dorsale de l'anneau.

Ainsi, l'anneau 1 peut présenter au repos, et notamment au moment de son implantation, une structure particulièrement compacte.

De façon particulièrement préférentielle, le second tronçon plissé 21 présente deux plis agencés selon un « S » couché contre la bande souple 2, de telle sorte que le premier organe d'appui 5 est sensiblement divisé, lorsqu'il se trouve au repos en configuration contractée, en une cavité principale de remplissage 5A et une sous-cavité de réserve 5B, cette dernière étant de volume initialement moindre que la cavité principale 5A et communiquant librement avec celle-ci.

Il est remarquable que, selon la variante de réalisation préférentielle illustrée sur les figures 2 et 3, le premier organe d'appui 5 présente une structure particulièrement simple, légère et compacte, qui peut notamment être facilement obtenue par moulage.

Bien entendu, la première membrane 10 est réalisée dans un matériau suffisamment souple pour autoriser son passage de la configuration contractée à la configuration déployée, tel qu'un matériau élastomère biocompatible du genre silicone.

De préférence, l'anneau 1 comporte également un second organe d'appui 105 conçu pour venir en appui contre l'organe biologique et apte à s'étendre vers l'intérieur dudit anneau 1 selon une seconde direction D2 sensiblement radiale, ledit second organe d'appui 105 étant délimité par une seconde membrane 110 pourvue d'une structure déformable qui est asymétrique vis-à-vis de le seconde direction D2.

Avantageusement, les caractéristiques du second organe d'appui 105, sa forme, son agencement, et son fonctionnement dynamique peuvent être déduits *mutatis mutandis* des caractéristiques du premier organe d'appui 5.

Par commodité de description, les références associées aux éléments correspondant à ce second organe d'appui 105 correspondront aux références associées aux éléments homologues du premier organe d'appui 5, incrémentées d'une valeur 100.

Ainsi, en particulier, le second organe d'appui 105 est de préférence formé par une seconde poche gonflable délimitée par une seconde membrane 110, laquelle peut être divisée en un premier tronçon lisse 120 raccordé à un second tronçon plissé 121 qui présente une réserve de matière 114 d'un côté du plan P2 médian audit second organe d'appui 105, lorsque ce dernier se trouve en configuration contractée.

De façon particulièrement préférentielle, tel que cela est illustré sur les figures 2 et 3, et selon une caractéristique qui peut constituer une invention à part entière, le premier organe d'appui 5 et le second organe d'appui 105 sont montés en opposition l'un par rapport à l'autre sur la structure porteuse 2, de telle sorte que la première direction D1 et la seconde direction D2 sont sensiblement parallèles et que lesdits premier et second organes d'appui 5, 105 peuvent s'étendre conjointement tandis que leurs structures déformables respectives agissent de manière sensiblement antagoniste.

Plus particulièrement, le premier et le second organe d'appui 5, 105 sont avantageusement agencés de telle sorte qu'ils peuvent suivre des expansions radiale sensiblement parallèles et de même sens, tandis que, en revanche, la tendance à la déviation latérale de la première membrane 10 s'exerce dans un sens transverse sensiblement opposé à celui de la déviation de la seconde membrane 110.

Ainsi, si la première membrane 10 tend à basculer de la droite vers la gauche lors du déploiement, tel que cela est illustré dans la vue en section de la figure 3, la seconde membrane 110 se déploie en revanche de la gauche vers la droite.

Plus particulièrement, les première et seconde poches gonflables 5, 105 présentent de préférence leurs réserves de matière 14, 114 respectives face à face, de telle sorte que leurs contributions sont sensiblement opposées.

Par ailleurs, de façon particulièrement préférentielle le premier et le second organe d'appui 5, 105 sont formés tous deux par des poches annulaires qui sont étagées selon l'axe d'extension (X-X') et qui s'étendent sensiblement parallèlement l'une à l'autre.

Ainsi, avantageusement, l'anneau 1 conforme à l'invention présente avec l'organe biologique une pluralité de zones de contact sensiblement annulaires et disjointes l'une de l'autre, et plus particulièrement superposée axialement à distance l'une de l'autre, ce qui lui confère une excellente stabilité.

Plus particulièrement, le premier et le second organe d'appui 5, 105 peuvent être séparés par une dépression 25 ouverte vers l'intérieur de l'anneau 1 et qui s'étend de préférence sur toute la longueur de la bande souple 2 de telle sorte que chacun des organes d'appui 5, 105 fournit un ancrage indépendant de type cerclage autour de l'organe biologique.

Avantageusement, chaque point d'ancrage est ainsi susceptible de retenir l'anneau en position même en cas de perte temporaire d'adhérence de l'autre point d'ancrage, par exemple sous l'effet des contractions péristaltiques de l'estomac.

En outre, cette séparation physique des zones de contact permet également d'obtenir une portée plus importante tout en réduisant la superficie effective desdites zones de contact entre l'anneau et l'organe biologique, ce qui limite les risques de lésion de la paroi stomacale, ainsi que la gêne causée au patient par l'anneau 1.

De façon particulièrement préférentielle, le premier organe d'appui 5 et le second organe d'appui 105 sont images l'un de l'autre par symétrie plane, en l'occurrence vis-à-vis du plan sagittal π de l'anneau 1 qui coupe la bande souple 2 formant la structure porteuse en son milieu.

Avantageusement, ledit plan sagittal π s'étend parallèlement aux plans médians P1, P2 respectifs desdits premier et second organe d'appui 5, 105 et sensiblement à mi-distance entre ces derniers.

De façon particulièrement avantageuse, la disposition en opposition du premier et du second organe d'appui 5, 105, en particulier lorsque ces derniers présentent des structures images l'une de l'autre, permet de rétablir la symétrie globale de l'anneau 1, ainsi que de son fonctionnement, par rapport au plan sagittal.

En d'autres termes, même si la structure individuelle d'un organe d'appui est localement asymétrique, la réunion de ces deux organes d'appui rétablit la symétrie de l'ensemble, ce qui évite tout déséquilibre lors du réglage et du maintien de l'anneau.

Par ailleurs, il est remarquable que, au sens de l'invention, le premier et le second organe d'appui 5, 105 peuvent être formés respectivement par une première et une seconde poche gonflable qui communiquent l'une avec l'autre et forment les deux portions saillantes d'une même chambre.

Selon une telle variante de réalisation, la chambre forme un contenant commun pour le fluide de remplissage et présente deux excroissances distinctes qui constituent lesdites poches de serrage 5, 105, et plus particulièrement présente une première et une seconde portion de crête faisant saillie vers l'intérieur de la boucle formée par l'anneau et destinées à venir au contact de l'organe biologique respectivement au niveau d'une première et d'une seconde zone de contact.

Avantageusement, la première et la seconde poche de gonflage 5, 105 peuvent ainsi être alimentées par un même conduit 6 qui les relie au niveau d'un collecteur commun.

Selon une variante de réalisation préférentielle, le premier et le second organe d'appui 5, 105 sont reliés l'un à l'autre par un pont 30 distinct de la structure porteuse 2.

Ainsi, ledit pont 30 forme un lien cinématique qui joint la première poche gonflable 5 à la seconde poche gonflable 105 en enjambant une zone vide de matière formant une cavité 31 qui sépare la première poche gonflable de la seconde poche gonflable.

Avantageusement, l'espace laissé vacant par la cavité 31 et délimité entre la première et la second poche gonflable 5, 105 par le pont 30 et la bande souple 2, forme une zone de stockage dans laquelle peuvent se replier la première et la seconde membrane 10, 110 lorsque l'anneau se trouve en configuration contractée.

De préférence, la cavité 31 se trouve en communication libre avec l'extérieur de l'anneau 1, au moyen d'un ou plusieurs orifices quelconques, par exemple ouverts aux extrémités de la bande souple 2. Ainsi, ladite cavité 31 est de préférence agencée pour ne pas pouvoir contenir ou retenir un fluide sous pression, et notamment un liquide, et est capable de s'adapter spontanément et passivement aux déformations des membranes 10, 110.

En outre, le pont 30 forme avantageusement un moyen de retenue cinématique qui limite le débattement de la première poche gonflable 5 par rapport à la seconde poche gonflable 105, et plus précisément le dégagement latéral antagoniste des première et seconde membranes 10, 110, lors de l'expansion radiale conjointe de ces dernières.

Ainsi, le pont 30 permet avantageusement d'équilibrer l'expansion latérale desdites membranes, en maintenant le premier et le second organe d'appui 5, 105 sensiblement parallèle l'un à l'autre, ces derniers se retenant mutuellement, de telle sorte que les mouvements latéraux antagonistes de leurs membranes respectives 10, 110 se compensent sensiblement.

En d'autres termes, le pont 30 évite le débordement excessif de l'une ou l'autre des poches gonflables d'un côté ou de l'autre de l'anneau, en limitant le basculement de leurs tronçons lisses 20, 120 respectifs, ce qui assure un développement régulier et groupé de l'ensemble formé par le premier et le second organe d'appui 5, 105.

De préférence, les attaches du tablier du pont 30 correspondent aux zones de raccordement entre le premier tronçon lisse 20, 120 et le second tronçon plissé 21, 121 de chacune des première et seconde membranes 10, 110.

Par ailleurs, le pont 30 peut avantageusement présenter une zone d'inflexion, par exemple en forme de V dont la pointe est orientée vers la bande souple 2, tel que cela est illustré sur la figure 3, de sorte à pouvoir se déformer élastiquement à la manière d'une charnière et ainsi s'adapter aux variations de la distance qui sépare la première membrane 10 et la seconde membrane 110 lors du déploiement ou de la contraction radiale de ces dernières.

Le fonctionnement d'un anneau conforme à celui représenté sur les figures 1 à 3 va maintenant être décrit.

Tout d'abord, le praticien introduit l'anneau ouvert, par exemple par laparoscopie, et l'engage autour de l'organe biologique.

Il incurve alors la structure porteuse 2 jusqu'à refermer celle-ci sur elle-même pour former une boucle enserrant l'organe biologique, et verrouille la structure porteuse 2 au niveau de ses extrémités par l'engagement de l'ergot 3 contre le manchon 4.

Ce faisant, les portions de crête du premier et du second organe d'appui 5, 105 viennent s'appliquer en appui contre la paroi de l'organe biologique, au niveau respectivement d'une première et d'une seconde zone de contact sensiblement annulaires et situées à distance le long de l'axe d'extension (X-X').

Au repos, c'est-à-dire avant le remplissage des première et seconde poches gonflables constituant le premier et le second organe d'appui 5, 105, lesdites poches se trouvent en configuration contractée, tel que cela est représenté en traits pleins sur la figure 3.

Plus particulièrement, ce sont les dômes saillants formés par les premiers tronçons lisses 20, 120 respectifs des première et seconde membranes 10, 110 qui présentent alors un profil bombé sensiblement hémicylindrique et dont les lignes de crête viennent au contact de l'organe biologique.

De préférence, dans une section de l'anneau le point culminant appartenant à la ligne de crête de chaque premier tronçon lisse 20, 120, c'est-à-dire le point de la surface externe de la membrane 10, 110 le plus proche de l'axe d'extension (X-X'), est situé sensiblement à l'aplomb de la première zone de jonction latérale 11, 111 de l'organe d'appui correspondant.

Il est remarquable que le volume de la cavité 31 est alors particulièrement réduit, le pont 30 se trouvant à proximité immédiate, voire au contact partiel de la structure porteuse 2.

Lorsque le praticien procède au gonflage des première et seconde poches gonflables 5, 105, c'est-à-dire lorsqu'il introduit un fluide de remplissage approprié dans celle-ci, l'augmentation de pression et/ou de volume qui résulte de cette injection force le déploiement radial centripète desdites poches gonflables.

Ce gonflage se traduit par un déploiement progressif et sensiblement simultané des première et seconde membranes 10, 110, de telle sorte que leur portion de crête s'éloigne progressivement de la structure porteuse 2 selon respectivement une première et une seconde direction D1, D2 sensiblement radiales, centripètes et parallèles entre elles.

Cette progression globalement radiale centripète s'accompagne d'un basculement des premiers tronçons lisses 20, 120 qui défléchissent vers les bords latéraux libres de l'anneau 1, tel que cela est illustré par les flèches sur la figure 3, de telle sorte que lesdits premiers tronçons lisses 20, 120 se redressent progressivement, en voyant leur rayon de courbure augmenter et en s'éloignant progressivement du plan sagittal π de l'anneau.

Ainsi, selon une caractéristique qui peut constituer une invention à part entière, chaque structure déformable conforme à l'invention est préférentiellement agencée pour provoquer le basculement de la membrane 10, 110 respective lors du déploiement radial centripète de cette dernière.

Plus particulièrement, le premier et le second organe d'appui 5, 105 sont donc de préférence agencés de telle sorte que leur déploiement radial centripète s'accompagne d'un déroulement de leurs membranes 10, 110 respectives vers le bord latéral de l'anneau qui leur est le plus proche.

Avantageusement, ce déroulement respectif de chacune des poches vers le bord latéral de l'anneau qui lui est le plus proche tend à limiter le déplacement relatif de la membrane 10, 110 par rapport à la paroi de l'organe biologique, et à provoquer notamment un déplacement relatif par roulement plutôt que par friction, de telle sorte que le déploiement est particulièrement respectueux des tissus dudit organe biologique.

En référence à la figure 3, la première membrane 10 tend à basculer globalement de la droite vers la gauche, son premier tronçon 20 se décalant latéralement en franchissant peu à peu le plan médian P1, tandis que la seconde membrane 110 tend à suivre une trajectoire sensiblement opposée, de la gauche vers la droite.

Avantageusement, l'appel de matière créé par ce dégagement latéral est compensé simultanément par le dépliage progressif des seconds tronçons plissés 21, 121 qui s'allongent en ouvrant peu à peu les plis constitutifs de leurs réserves de matière 14, 114.

Le déploiement peut avantageusement se poursuivre jusqu'à la résorption des réserves de matière 14, 114, c'est-à-dire jusqu'à redressement des seconds tronçons 21, 121 initialement plissés.

A ce titre, il est remarquable que les réserves de matière 14, 114 permettent avantageusement de déployer les poches gonflables 5, 105 sans avoir à distendre, et par conséquent à fragiliser, leurs membranes 10, 110 respectives, ce qui augmente la résistance et la longévité de l'anneau.

Avantageusement, le pont 30 maintient un lien cinématique transverse entre lesdites première et seconde membranes 10, 110 et oppose par conséquent un effort de retenue mutuelle au libre débattement axial, et plus particulièrement à l'écartement, sensiblement selon un mouvement d'ouverture en corolle, des première et seconde poches gonflables 5, 105 selon l'axe d'extension (X-X').

En d'autres termes, le pont 30 autorise avantageusement le déroulement antagoniste des première et seconde membranes 10, 110, tout en maintenant le premier et le second organe d'appui 5, 105 sensiblement groupés et parallèles lors de leur déploiement radial, ce qui assure un déploiement régulier de l'anneau 1 jusqu'à ce que celui-ci atteigne sa configuration de constriction fonctionnelle, et par exemple sa configuration de déploiement maximal illustrée en pointillés sur la figure 3.

Par ailleurs, il est remarquable que lors du déploiement des première et seconde membranes 10, 110, le pont 30 « décolle » de la structure porteuse 2 et est entraîné en déplacement radial centripète par la déformation desdites première et seconde membranes 10, 110 sans opposer de résistance significative à ce mouvement.

Par ailleurs, le pont 30 autorise avantageusement, notamment grâce à sa zone d'inflexion flexible, un débattement axial juste nécessaire et suffisant au bon déploiement des poches gonflables et peut en particulier s'adapter à tout moment à la largeur résiduelle de la dépression 25 qui subsiste entre lesdites poches.

En outre, ledit pont 30 reste avantageusement en retrait des portions de crête des organes d'appui 5, 105, en particulier lorsque ceux-ci sont déployés au maximum, c'est-à-dire au plus près de l'axe d'extension (X-X'), de telle sorte que l'ancrage double de l'anneau 1 est toujours opérationnel et conserve à ce dernier une bonne stabilité.

Bien entendu, tous les éléments de description qui ont été fournis dans le cadre d'un déploiement radial centripète des organes d'appui 5, 105 restent applicables, *mutatis mutandis,* à la contraction radiale centrifuge desdits organes d'appui 5, 105.

Ainsi, lorsque le praticien dégonfle les poches gonflables 5, 105 de sorte à les faire revenir de leur configuration déployée à leur configuration contractée, c'est-à-dire de sorte à augmenter le diamètre de constriction de l'organe biologique, les portions de crête desdites poches s'affaissent en *« redescendant »* vers la structure porteuse 2, selon un mouvement radial centrifuge et entraînent à leur suite le pont 30, ce qui a pour effet d'écraser progressivement la cavité 31.

Plus particulièrement, les premiers tronçons 20, 120 respectifs des poches de gonflage 5, 105 fléchissent et basculent pour se rapprocher du plan sagittal π, en (re)traversant en sens inverse les plans médians P1, P2 des première et seconde poches gonflables 5, 105, selon un mouvement d'enroulement, tandis que les seconds tronçons absorbent progressivement le surplus de matière en se plissant.

A ce titre, l'élasticité intrinsèque des seconds tronçons 21, 121 confère avantageusement une mémoire de forme aux première et seconde membranes 10, 110.

D'autre part, lors de cet affaissement, le pont 30 agit comme un ressort de rappel qui tend à rapprocher l'une de l'autre les première et seconde membranes 10, 110 situées en vis-à-vis l'une de l'autre de part et d'autre de la dépression 25.

Avantageusement, ces effets peuvent se combiner pour faciliter le pliage ordonné et progressif desdits seconds tronçons 21, 121 jusqu'à la reconstitution des réserves de matière 14, 114 en configuration contractée, les première et seconde membranes 10, 110 se repliant progressivement pour se *« ranger »* sous leurs premiers tronçons 20, 120 respectifs, ce qui permet *« d'entreposer »* lesdites réserves de matière 14, 114 sensiblement au centre de la bande souple.

Il est remarquable que, de nouveau, ni le pont 30 ni la cavité 31 n'opposent de résistance significative à ce mouvement radial centrifuge, de telle sorte que les première et seconde poches de serrage 5, 105 peuvent regagner, notamment par retour élastique, toute position intermédiaire jusqu'à leur position contractée initiale.

Ainsi, l'anneau 1 conforme à l'invention présente un comportement dynamique particulièrement respectueux de l'organe biologique, puisqu'il limite le frottement contre la paroi dudit organe biologique, tout en évitant le pincement de ladite paroi, aussi bien lors du déploiement que de la contraction des organes d'appui 5, 105.

En outre, le passage de la configuration contractée à la configuration déployée, ou inversement, est avantageusement régulier et reproductible, les membranes suivant des trajectoires sensiblement prévisibles, contrôlées et réversibles.

Par ailleurs, l'anneau 1 conforme à l'invention présente une structure ajourée particulièrement légère et robuste qui est de surcroît peu soumise au travail en fatigue, de telle sorte que l'anneau 1 possède une importante longévité.

Enfin, il est remarquable que l'anneau 1 conforme à l'invention permet un réglage précis, continu et fin de la constriction, tout en conservant en permanence ses qualités de confort et de stabilité dynamique.

### MEILLEURE MANIERE DE REALISER L'INVENTION

L'invention trouve son application industrielle dans la conception et la fabrication d'anneaux chirurgicaux, et en particulier d'anneaux gastriques.

## Revendications

1. Anneau chirurgical (1) implantable destiné à être placé autour d'un organe biologique constituant une poche ou un conduit, en s'enroulant autour d'un axe moyen d'extension (X-X'), en vue de modifier la section de passage dudit organe biologique, ledit anneau comportant au moins un premier organe d'appui (5) conçu pour venir en appui contre l'organe biologique et apte à s'étendre vers l'intérieur de l'anneau (1) selon une première direction (D) sensiblement radiale, ledit anneau étant **caractérisé en ce que** ledit premier organe d'appui (5) est délimité par une première membrane (10) pourvue d'une structure déformable qui est asymétrique vis-à-vis de ladite première direction (D1) et qui est conçue pour provoquer, lors de l'expansion du premier organe d'appui (5) selon la première direction (D1) sensiblement radiale, la migration conjointe d'au moins une partie de la première membrane (10) d'un côté à l'autre d'un plan (P1) médian audit premier organe d'appui (5) et normal à l'axe d'extension (X-X').

2. Anneau chirurgical selon la revendication 1 **caractérisé en ce que** la structure déformable est agencée pour provoquer le basculement latéral de la membrane (10) lors du déploiement radial centripète de cette dernière.

3. Anneau chirurgical selon la revendication 1 ou 2 **caractérisé en ce qu'**il comporte une structure porteuse (2) à laquelle le premier organe d'appui (5) est fixé respectivement au niveau d'une première zone de jonction latérale (11) et d'une seconde zone de jonction latérale (12) distantes l'une de l'autre, et **en ce que** la première membrane (10) est rabattue sur elle-même du côté de la première zone de jonction latérale (11), lorsque le premier organe d'appui (5) se trouve en configuration contractée, pour former une réserve de matière (14) située sensiblement à l'opposé de la seconde zone de jonction latérale (12).

4. Anneau chirurgical selon la revendication 3 **caractérisé en ce que** la première membrane (10) comprend, lorsque le premier organe d'appui (5) se trouve en configuration contractée, un premier tronçon (20) dit " lisse " relié à la seconde zone de jonction latérale (12) et un second tronçon (21) dit " plissé " relié à la première zone de jonction latérale (11), le premier tronçon lisse (20) rejoignant le second tronçon plissé (21) et formant un dôme qui recouvre à la fois ledit second tronçon plissé (21) et la première zone de jonction latérale (11).

5. Anneau chirurgical selon l'une des revendications précédentes **caractérisé en ce qu'**il comporte un second organe d'appui (105) conçu pour venir en appui contre l'organe biologique et apte à s'étendre vers l'intérieur de l'anneau selon une seconde direction (D2) sensiblement radiale, ledit second organe d'appui étant délimité par une seconde membrane (110) pourvue d'une structure déformable qui est asymétrique vis-à-vis de ladite seconde direction (D2).

6. Anneau chirurgical selon la revendication 5 **caractérisé en ce qu'**il comporte une structure porteuse (2) sur laquelle le premier et le second organe d'appui (5, 105) sont montés en opposition, de telle sorte que la première direction (D1) et la seconde direction (D2) sont sensiblement parallèles et que lesdits premier et second organe d'appui (5, 105) peuvent s'étendre conjointement, tandis que leurs structures déformables respectives agissent de manière sensiblement antagoniste.

7. Anneau chirurgical selon la revendication 6 **caractérisé en ce que** le premier et le second organe d'appui (5, 105) sont reliés l'un à l'autre par un pont (30) distinct de la structure porteuse (2).

8. Anneau chirurgical selon l'une des revendications 5 à 7 **caractérisé en ce que** le premier et le second organe d'appui (5, 105) sont formés par des poches annulaires qui sont étagées selon l'axe d'extension (X-X') et qui s'étendent sensiblement parallèlement l'une à l'autre.

9. Anneau chirurgical selon la revendication 8 **caractérisé en ce que** le premier et le second organe d'appui (5, 105) sont agencés de telle sorte que leur déploiement radial centripète s'accompagne d'un déroulement de leur membrane (10, 110) respective vers le bord latéral de l'anneau qui leur est le plus proche.

10. Anneau chirurgical selon l'une des revendications 5 à 9 **caractérisé en ce que** le premier organe d'appui (5) et le second organe d'appui (105) sont images l'un de l'autre par symétrie plane.

11. Anneau chirurgical selon l'une des revendications précédentes **caractérisé en ce qu'**il constitue un anneau de gastroplastie destiné au traitement de l'obésité.

12. Anneau chirurgical selon l'une quelconque des revendications précédentes, dans lequel le premier organe d'appui (5) présente une structure sensiblement annulaire d'axe (X-X').

13. Anneau chirurgical selon les revendications 3 et 12, dans lequel le premier organe d'appui s'étend sensiblement sur toute la longueur de la structure porteuse (2)

14. Anneau chirurgical selon la revendication 13, dans lequel le premier organe d'appui (5) comporte un premier tronçon (20) destiné à venir en contact avec ledit organe biologique, ce premier tronçon formant un dôme régulier à la surface lisse et convexe par rapport à l'intérieur de l'anneau (1).

## Patentansprüche

1. Implantierbarer chirurgischer Ring (1) zum Legen um ein biologisches Organ herum, das eine Mulde oder einen Kanal bildet, indem er sich um eine mittlere Erstreckungsachse (X-X') windet, um den Durchlassquerschnitt des biologischen Organs zu ändern, wobei der Ring mindestens ein erstes Stützelement (5) umfasst, das ausgelegt ist, um sich an dem biologischen Organ abzustützen, und in der Lage ist, sich zum Innern des Rings (1) in einer ersten im Wesentlichen radialen Richtung (D1) zu erstrecken, wobei der Ring **dadurch gekennzeichnet ist, dass** das erste Stützelement (5) durch eine erste Membran (10) abgegrenzt ist, die mit einer verformbaren Struktur versehen ist, die gegenüber der ersten Richtung (D1) asymmetrisch ist und die ausgelegt ist, um bei der Ausdehnung des ersten Stützorgans (5) in der ersten im Wesentlichen radialen Richtung (D1) die gemeinsame Verlagerung mindestens eines Teils der ersten Membran (10) von einer Seite zur anderen Seite einer Ebene (P1), die zu dem ersten Stützelement (5) mittig und zu der Erstreckungsachse (X-X') senkrecht ist, zu verursachen.

2. Chirurgischer Ring nach Anspruch 1, **dadurch gekennzeichnet, dass** die verformbare Struktur angeordnet ist, um das seitliche Verkippen der Membran (10) bei der zentripetalen radialen Entfaltung derselben zu verursachen.

3. Chirurgischer Ring nach Anspruch 1 oder 2, **dadurch gekennzeichnet, dass** er eine Trägerstruktur (2) umfasst, an der das erste Stützelement (5) jeweils an einer ersten seitlichen Verbindungszone (11) und einer zweiten seitlichen Verbindungszone (12), die voneinander beabstandet sind, befestigt ist, und dass die erste Membran (10) auf der Seite der ersten seitlichen Verbindungszone (11) in sich eingeklappt ist, wenn sich das erste Stützorgan (5) in der zusammengezogenen Konfiguration befindet, um eine Materialreserve (14) zu bilden, die sich im Wesentlichen gegenüber der zweiten seitlichen Verbindungszone (12) befindet.

4. Chirurgischer Ring nach Anspruch 3, **dadurch gekennzeichnet, dass** die erste Membran (10), wenn sich das erste Stützelement (5) in der zusammengezogenen Konfiguration befindet, ein erstes so genanntes "glattes" Teilstück (20), das mit der zweiten seitlichen Verbindungszone (12) verbunden ist, und ein zweites so genanntes "faltiges" Teilstück (21), das mit der ersten seitlichen Verbindungszone (11) verbunden ist, umfasst, wobei das erste glatte Teilstück (20) auf das zweite faltige Teilstück (21) trifft und eine Kuppe bildet, die sowohl das zweite faltige Teilstück (21) als auch die erste seitliche Verbindungszone (11) abdeckt.

5. Chirurgischer Ring nach einem der vorhergehenden Ansprüche, **dadurch gekennzeichnet, dass** er ein zweites Stützorgan (105) umfasst, das ausgelegt ist, um sich an dem biologischen Organ abzustützen, und in der Lage ist, sich zum Innern des Rings in einer zweiten im Wesentlichen radialen Richtung (D2) zu erstrecken, wobei das zweite Stützelement, das von einer zweiten Membran (110) abgegrenzt ist, mit einer verformbaren Struktur versehen ist, die gegenüber der zweiten Richtung (D2) asymmetrisch ist.

6. Chirurgischer Ring nach Anspruch 5, **dadurch gekennzeichnet, dass** er eine Trägerstruktur (2) umfasst, auf der das erste und das zweite Stützelement (5, 105) gegenüberliegend installiert sind, so dass die erste Richtung (D1) und die zweite Richtung (D2) im Wesentlichen parallel sind, und dass sich die ersten und zweiten Stützelemente (5, 105) zusammen erstrecken können, während ihre jeweiligen verformbaren Strukturen im Wesentlichen entgegengesetzt wirken.

7. Chirurgischer Ring nach Anspruch 6, **dadurch gekennzeichnet, dass** das erste und das zweite Stützelement (5, 105) über eine Brücke (30), die sich von der Trägerstruktur (2) unterscheidet, miteinander verbunden sind.

8. Chirurgischer Ring nach einem der Ansprüche 5 bis 7, **dadurch gekennzeichnet, dass** das erste und das zweite Stützelement (5, 105) durch ringförmige Mulden gebildet sind, die entlang der Erstreckungsachse (X-X') abgestuft sind und die sich im Wesentlichen parallel zueinander erstrecken.

9. Chirurgischer Ring nach Anspruch 8, **dadurch gekennzeichnet, dass** das erste und das zweite Stützelement (5, 105) derart angeordnet sind, dass ihre zentripetale radiale Entfaltung mit einem Abrollen ihrer jeweiligen Membran (10, 110) in Richtung auf den seitlichen Rand des Ringes, der ihnen am nächsten liegt, einhergeht.

10. Chirurgischer Ring nach einem der Ansprüche 5 bis 9, **dadurch gekennzeichnet, dass** das erste Stützelement (5) und das zweite Stützelement (105) plansymmetrische Abbilder voneinander sind.

11. Chirurgischer Ring nach einem der vorhergehenden Ansprüche, **dadurch gekennzeichnet, dass** er einen Gastroplastikring bildet, der zur Behandlung von Fettleibigkeit gedacht ist.

12. Chirurgischer Ring nach einem der vorhergehenden Ansprüche, wobei das erste Stützelement (5) eine im Wesentlichen ringförmige Struktur mit einer Achse (X-X') aufweist.

13. Chirurgischer Ring nach Anspruch 3 und 12, wobei sich das erste Stützelement im Wesentlichen über die ganze Länge der Trägerstruktur (2) erstreckt.

14. Chirurgischer Ring nach Anspruch 13, wobei das erste Stützelement (5) ein erstes Teilstück (20) umfasst, das dazu gedacht ist, mit dem biologischen Organ in Berührung zu kommen, wobei dieses erste Teilstück eine gleichförmige Kuppe mit glatter und im Verhältnis zum Innern des Rings (1) konvexer Oberfläche bildet.

## Claims

1. An implantable surgical ring (1) intended to be placed around a biological organ constituting a pocket or a duct, by winding around a mean axis of extension (X-X') in view of modifying the section of passage of said biological organ, said ring comprising at least one first bearing member (5) designed to press against the biological organ and being capable of extending into the interior of the ring (1) along a first substantially radial direction (D1), said ring being **characterized in that** said first bearing member (5) is delimited by a first membrane (10) provided with a deformable structure that is asymmetrical with regard to said first direction (D1) and that is designed, during expansion of the first bearing member (5) along the first substantially radial direction (D1), to bring about the joint migration of at least one part of the first membrane (10) from one side to the other in a median plane (P1) of said first bearing member (5) normal to the axis of extension (X-X').

2. The surgical ring according to claim 1, **characterized in that** the deformable structure is configured to bring about lateral tilting of the membrane (10) during the centripetal radial deployment of this membrane.

3. The surgical ring according to claim 1 or 2, **characterized in that** the ring comprises a bearing structure (2) to which the first bearing member (5) is fixed respectively at the level of a first lateral junction zone (11) and a second lateral junction zone (12) which are distant from each other, and **in that** the first membrane (10) is folded down on itself on the first lateral junction zone (11) side when the first bearing member (5) is in a contracted configuration, to form a reserve of material (14) situated substantially opposite the second lateral junction zone (12).

4. The surgical ring according to claim 3, **characterized in that** the first membrane (10) comprises, when the first bearing member (5) is in a contracted configuration, a first "smooth" section (20) connected to the second lateral junction zone (12) and a second "folded" section (21) connected to the first lateral junction zone (11), the first smooth section (20) meeting the second folded section (21) and forming a dome which covers at the same time said second folded section (21) and the first lateral junction zone (11).

5. The surgical ring according to one of the previous claims, **characterized in that** the ring comprises a second bearing member (105) designed to press against the biological organ and is capable of extending into the interior of the ring along a second substantially radial direction (D2), said second bearing member being defined by a second membrane (110) provided with a deformable structure which is asymmetrical with regard to the second direction (D2).

6. The surgical ring according to claim 5, **characterized in that** the ring comprises a bearing structure (2) on which the first and second bearing members (5, 105) are mounted in opposition to each other such that the first direction (D1) and the second direction (D2) are substantially parallel and **in that** said first and second bearing members (5, 105) may extend jointly while their respective deformable structures act in a substantially antagonistic manner.

7. The surgical ring according to claim 6, **characterized in that** the first and second bearing members (5, 105) are connected to each other by a bridge (30) distinct from the bearing structure (2).

8. The surgical ring according to one of claims 5 to 7 **characterized in that** the first and second bearing members (5, 105) are formed by ring-shaped pockets that are stacked along the extension axis (X-X') and that extend substantially parallel to each other.

9. The surgical ring according to claim 8, **characterized in that** the first and second bearing members (5, 105) are arranged such that their centripetal radial deployment is accompanied by an unrolling of their respective membranes (10, 110) towards the lateral edge of the ring which is closest to each of them.

10. The surgical ring according to one of claims 5 to 9 **characterized in that** the first bearing member (5) and the second bearing member (105) are images of each other by planar symmetry.

11. The surgical ring according to any one of the previous claims **characterized in that** the ring constitutes a gastroplasty ring designed for the treatment of obesity.

12. The surgical ring according to any one of the previous claims, in which the first bearing member (5) presents a substantially ring-shaped structure with the axis (X-X').

13. The surgical ring according to claims 3 and 12, in which the first bearing member extends substantially over the entire length of the bearing structure (2).

14. The surgical ring according to claim 13, in which the first bearing member (5) comprises a first section (20) intended to come into contact with said biological organ, this first section forming a regular dome with a surface that is smooth and convex with relation to the interior of the ring (1).
